# EUROPEAN PATENT SPECIFICATION

(11) **EP 1 108 050 B1**
(45) Date of publication and mention of the grant of the patent: **06.10.2004**
(21) Application number: 99946642.8
(22) Date of filing: 26.08.1999
(51) Int. Cl.: C12N 15/86, A61K 48/00

(54) **METHODS AND COMPOSITIONS FOR EXPRESSING HETEROLOGOUS GENES IN HEPATOCYTES USING HEPADNAVIRAL VECTORS**
METHODEN UND ZUSAMMENSETZUNGEN FÜR DIE EXPRESSION VON HETEROLOGEN GENEN IN LEBERZELLEN UNTER VERWENDUNG VON VEKTOREN, DIE VON HEPADNAVIREN ABGELEITET SIND
TECHNIQUES ET COMPOSITIONS D'EXPRESSION DE GENES HETEROLOGUES DANS DES HEPATOCYTES PAR DES VECTEURS HEPADNAVIRAUX

(30) Priority: 26.08.1998 US 98173 P
(43) Date of publication of application: 20.06.2001
(73) Proprietor: Schaller, Heinz, 69120 Heidelberg (DE); Protzer, Ulrike, 69221 Dossenheim (DE); Nassal, Michael, 69181 Leimen (DE)
(72) Inventor: Schaller, Heinz, 69120 Heidelberg (DE); Protzer, Ulrike, 69221 Dossenheim (DE); Nassal, Michael, 69181 Leimen (DE)
(74) Representative: Rudolph, Ulrike, Dr.
(86) International application number: PCT/US1999/019452
(87) International publication number: WO 2000/012739

(56) References cited:
- WO-A-98/09524
- US-A- 5 981 274
- CHAISOMCHIT S. ET AL.: "Development of replicative and nonreplicative hepatitis B virus vectors." GENE THERAPY, vol. 4, no. 12, 1997, pages 1330-1340, XP000874038
- GUTTERMAN J. U.: "Cytokine therapeutics: Lessons from interferon alpha." PROC. NATL. ACAD. SCI. U.S.A., vol. 91, 1994, pages 1198-1205, XP002129420
- KONISHI K. ET AL.: "A simple and sensitive bioassay for the detection of human interleukin-18/interferon-y-inducing factor using human myelomonocytic KG-1 cells" JOURNAL OF IMMUNOLOGICAL METHODS, vol. 209, no. 2, 1 January 1997 (1997-01-01), pages 187-191, XP004103796 ISSN: 0022-1759
- PROTZER U. ET AL.: "INTERFERON GENE TRANSFER BY A HEPATITIS B VIRUS VECTOR EFFICIENTLY SUPPRESSES WILD-TYPE VIRUS INFECTION" PROCEEDINGS OF THE NATIONAL ACADEMY OF SCIENCES OF THE UNITED STATES OF AMERICA, vol. 96, no. 19, 14 September 1999 (1999-09-14), pages 10818-10823, XP002129421

## Description

### Background of the Invention

Chronic hepatitis B is one of the most common and most severe viral infections known with the number of virus carriers estimated to exceed 350 million (Who (1996) *WHO Communicable*, 1:1-26). These individuals are at high risk of developing liver cirrhosis and, eventually, primary liver cell carcinoma (Lau *et al*., (1993) *Lancet* 342:1335-1340). While a vaccine is available, currently used systemic treatment of chronic infection with high doses of interferon alpha (IFNα) leads to HBeAg / anti-HBe seroconversion in about one third of treated patients, and to virus elimination in only 10-25% of treated patients (Hoofnagle *et al*. (1997) *J*. *Viral Hepat*. 1:41-50; Lau *et al*. (1997) *Gastroenterology* 113:1660-1667). Furthermore, the currently used treatment regimen is costly and often has side effects.

The causative agent of hepatitis B disease is the hepatitis B virus (HBV), a member of the hepadnaviruses family. These small, DNA-containing viruses replicate through reverse transcription, like retroviruses, but do not integrate into the host cell genome for replication. One characteristic property of the hepadnaviruses is their high species and tissue-specificity. HBV replicates only in the liver of humans and higher primates and infects *in vitro* only primary hepatocytes of these hosts.

To date, most gene transfer clinical trials have relied on recombinant retroviral or adenoviral vectors for gene transfer, although both retroviral or adenoviral technologies have limitations. For example, with adenoviral vectors, the high multiplicity of infection may result in cytotoxicity in the target cells (Kay *et al*., (1993) *Cell Biochem,* 17E, 207), and the host immune response against adenoviral late gene products, e.g. penton protein, cause an inflammation response and the destruction of the infected tissue which received the vectors (Yang *et al*., (1994) *Proc*. *Natl*. *Acad. Sci.* 92, 4407-4411). In the WO 98/09524, Srivastava et al. point out, that cytokines and other immunomodulators may be useful for treatment of hepatic diseases or defects when expressed by adeno associated virus (AAV) vectors.
With retroviral vectors, the murine leukemia virus (MLV) is most widely used.
However, it is difficult to produce a virus at a reasonable titre for targeting a specific cell type or tissue by direct i*n vivo* delivery with MLV-based vectors (Kasahara *et al*., (1994) *Science,* 266, 1373-1376), and MLV-vectors, when packaged in murine packaging lines, cannot deliver a gene of interest to non-dividing cells such as hepatocytes. A further disadvantage of both retroviral or adenoviral technologies results from their ability to infect a broad range of cell types. Therefore, these gene therapy vectors are not ideal for specific delivery of genes to hepatocytes.

Hepadnaviridae are small enveloped DNA-viruses that employ for genome replication a reverse transcription pathway without requiring integration into the host genome. As exemplified by their type-member, the human hepatitis B virus (HBV) which infects only hepatocytes of humans and hominoid primates, hepatitis B viruses are highly species- and tissue-specific, targeted to the liver, and capable of infecting non-dividing hepatocytes. Because of these properties, they have been considered as attractive candidates for therapeutic, liver-directed gene transfer.

Early studies in the duck hepatitis B virus (DHBV) animal model had suggested that this was principally possible, although an effective gene delivery system remains to be developed. DHBV genomes containing short deletions, or small insertions of noncoding DNA, could be complemented in trans to allow formation of infectious, defective DHBV particles (Horwich, A.L. et al. (1990) *J. Virol*. 64:542-550). However, these data also revealed severe constraints with respect to the insert size. More recent attempts to produce HBV recombinants were in keeping with these observations: even insertion of a small marker gene (HIV-1 tat, 276 bp) markedly reduced the yield of enveloped virus particles. Chaisomchit, S. et al. ((1997) *Gene Ther*. 4:1330-1340) describe the construction of a replication deficient human hepatitis B virus (HBV) wherein a heterologous gene is inserted in frame with the viral polymerase. The insertion site touches the relevant RC signal in the HBV genome, and due to the insertion cis-acting sequences are destroyed and most of the POL open reading frame of the HBV genome is deleted. Chaisomchit et al. were not able to show production of recombinant viral particles covered by an envelope and sufficient to mediate infection. Obviously Chaisomchit et al. did not obtain mature enveloped virus particles that are infectious. Particle titers they obtained were far below that which they obtained from parallel transfection of an HBV wildtyp genome and would not be sufficient for a gene transfer vector et all.
Insertion into various parts of the viral genome of an 800 bp cassette containing the neomycin resistance gene was found to require compensation by comparably sized deletions (Chiang, P.W. (1992) Thesis, University of Heidelberg). In either study, expression of the transgene could be demonstrated only in transient transfection experiments, but not via an infectious recombinant virus particle. Hence, there is at present no experimental evidence demonstrating the capability of hepadnaviruses to function as gene-transfer vectors.

In view of the foregoing, methods and compositions for delivering a heterologous gene (*e*.*g*., a therapeutic gene) to hepatocytes via infection with a recombinant hepadnaviral particle such that expression of the heterologous gene is achieved in the hepatocytes are still needed.

### Summary of the Invention

The invention provides methods and compositions for efficient, hepatocyte-specific delivery and expression of heterologous genes, both *in-vitro* and *in-vivo*, using hepadnaviral vectors.

Hepatitis B viruses are hepatotropic DNA viruses that replicate extrachromosomally. The current invention is based, at least in part, on experiments using the duck hepatitis B virus (DHBV) model, that demonstrate that recombinant hepadnaviruses are suitable for liver-directed gene transfer. Green fluorescent protein as an intracellular marker, and a type 1 Interferon as a secretory protein with therapeutic potential, were expressed in a hepatocyte-specific, dose-dependent fashion upon DHBV gene transfer. Cells with pre-existing DHBV infection could be superinfected with recombinant virus, and Interferon expression efficiently suppressed wild-type virus replication. Similar HBV vectors were prepared and also were effective for delivering heterologous genes to hepatocytes. Thus, HBV-based viral vectors offer a novel approach to the treatment of liver disorders including chronic viral infections.

In one aspect, the invention pertains to a method for expressing a heterologous gene in hepatocytes. The method involves:
providing replication defective hepadnavirus particles at a titre level competent to infect hepatocytes, wherein a region of at least about 200 nucleotides of the S-gene of the hepadnavirus genome has been replaced with a heterologous gene of about 200 up to about 1200 nucleotides such that expression of the heterologous gene is regulated by regulatory sequences of the S-gene; and
infecting hepatocytes with the hepadnavirus such that the heterologous gene is delivered into the hepatocytes and expressed in the hepatocytes.

Preferably, the replication defective hepadnavirus particles are human hepatitis B virus particles.

In one embodiment, the heterologous gene replaces the region of the S-gene such that nucleotides encoding at least one amino acid of the S protein are fused in-frame to the 5' end of the heterologous gene. In another embodiment, the heterologous gene replaces the sequence of the S-gene at a site equivalent to the KpnI site at position 1290 of duck hepadnavirus. In still another embodiment, the heterologous gene replaces a region of the S-gene at a site equivalent to the KpnI site at position 1290 of duck hepadnavirus, and the heterologous gene is inserted such that nucleotides encoding at least one amino acid of the S protein are fused in-frame to the 5' end of the heterologous gene. In yet another embodiment, the heterologous gene replaces a region of the S-gene, and the heterologous gene is inserted such that nucleotides encoding at least one amino acid of the S protein are fused in-frame to the 5' end of the heterologous gene. In yet another embodiment, the heterologous gene replaces the S-gene. In still another embodiment, the heterologous gene replaces the S-gene and at least part of the preS region.

Preferred heterologous genes for expression in hepatocytes are genes encoding modulating agents (i.e., agents that modulate a viral infection of the hepatocytes or other disorder of the hepatocytes). Preferred modulating agents are cytokines. A particularly preferred cytokine is IFNα (Type I IFN).

Another aspect of the invention pertains to a method of treating a subject with a hepatic disorder. The method involves:
providing replication defective hepadnavirus particles at a titre level competent to infect hepatocytes of the subject with the hepatic disorder, wherein a region of the S-gene of the hepadnavirus genome has been replaced with a therapeutic gene such that expression of the therapeutic gene is regulated by regulatory sequences of the preS/S-gene; and
infecting hepatocytes of the subject with the hepadnavirus particles such that the therapeutic gene is delivered into the hepatocytes and expressed in the hepatocytes at a level sufficient to treat the hepatic disorder.

Preferred hepatic disorders to be treated by the method include hepatitis B, hepatitis C, hepatocellular carcinoma, cirrhosis, steatosis, hemochromatosis, and inherited liver disorders.

Preferred therapeutic genes include genes encoding modulating agents, such as cytokines. Preferred cytokines include IFNα, IFNβ, IFNγ , IL-18 and TNFα.

In one embodiment, the hepadnavirus particle is directly administered to the subject. In another embodiment, the hepadnavirus construct and a helper virus construct are cultured *in vitro* and the infectious particles produced from the culture are administered to the subject. In another embodiment, recombinant hepadnavirus particles are produced by a helper cell line, and are administered to the subject.

The invention also provides a method of treating a subject with a hepatitis infection. The method involves:
providing replication defective hepadnavirus particles at a titre level competent to infect hepatocytes of the subject with hepatitis, wherein a region of the S-gene of the hepadnavirus genome has been replaced with a gene encoding a cytokine such that expression of the gene encoding a cytokine is regulated by regulatory sequences of the preS/S-gene; and
infecting hepatocytes of the subject with the hepadnavirus such that the gene encoding a cytokine is delivered to the hepatocytes and expressed in the hepatocytes at a level sufficient to treat the hepatitis.

Preferably, the cytokine is IFNα. Alternatively, the cytokine can be, for example, TNFα, IFNβ, IL-18 or IFNγ.

In a particularly preferred embodiment, the hepatitis infection is hepatitis B and the cytokine is IFNα.

Another aspect of the invention pertains to a replication defective hepadnavirus particle, wherein a region of at least about 200 nucleotides of the S-gene of the hepadnavirus genome has been replaced with a therapeutic gene (e.g., a cytokine gene, such as such INFα) of about 200 up to about 1200 nucleotides such that expression of the therapeutic gene is regulated by regulatory sequences of the S-gene. Pharmaceutical compositions comprising the replication defective hepadnavirus particle and a pharmaceutically acceptable carrier or the replication defective hepadnavirus particle and a helper virus are also encompassed by the invention.

Yet another aspect of the invention pertains to a method of producing therapeutic replication defective hepadnavirus particles at a titre level suitable for therapeutic use. The method involves:
co-transfecting cells of a hepatoma cell line with:
   (i) replication defective hepadnavirus constructs, wherein a region of at least about 200 nucleotides of the S-gene of the hepadnavirus DNA has been replaced with a gene of about 200 up to about 1200 nucleotides encoding a therapeutic gene such that expression of the gene encoding a cytokine is regulated by regulatory sequences of the S-gene; and
   (ii) a helper construct;
culturing these cells until infectious viral particles are produced; and recovering the infectious viral particles.

### Brief Description of the Drawings

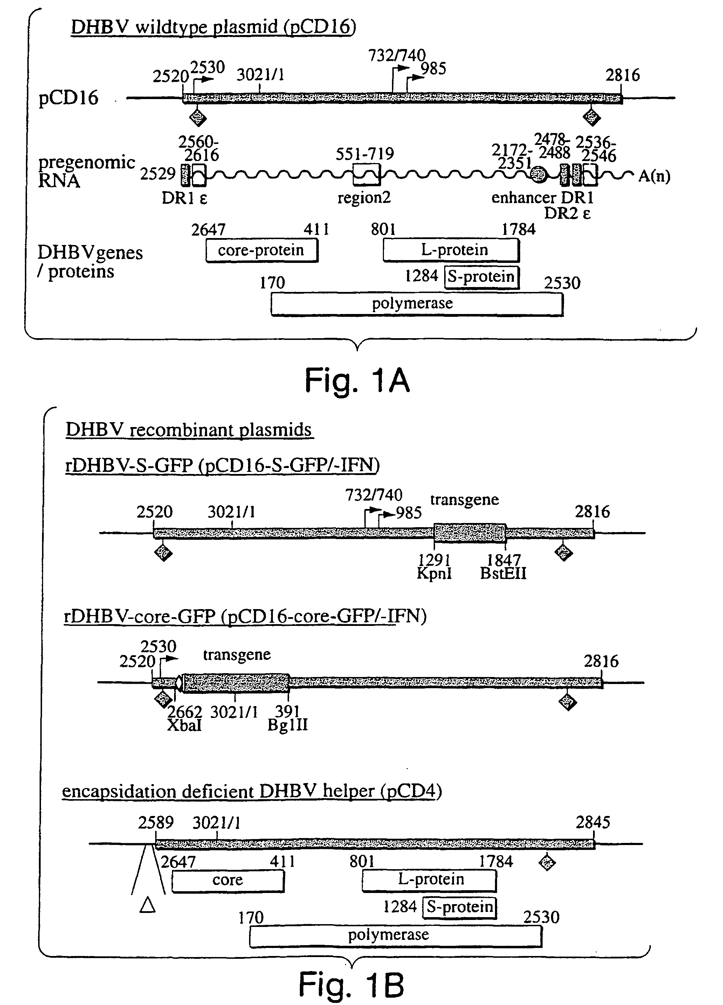

Figure 1A is a schematic diagram depicting the plasmid pCD16 expressing wild type DHBV, pregenomic DHBV-RNA displaying important cis-elements, and DHBV proteins.

Figure 1B is a schematic diagram depicting three DHBV recombinant transfer plasmids. In the first plasmid, rDHBV-S-GFP (rDHBV-S-GFP/IFN), the transgene replaces the S-gene of DHBV. In the second plasmid, rDHBV-core-GFP (rDHBV-core-GFP/IFN), the transgene replaces the core-gene of DHBV. The third plasmid is the pCD4 encapsidation deficient DHBV helper plasmid. Viral gene products lacked by the first and second plasmids are provided by cotransfection of the third, helper plasmid.

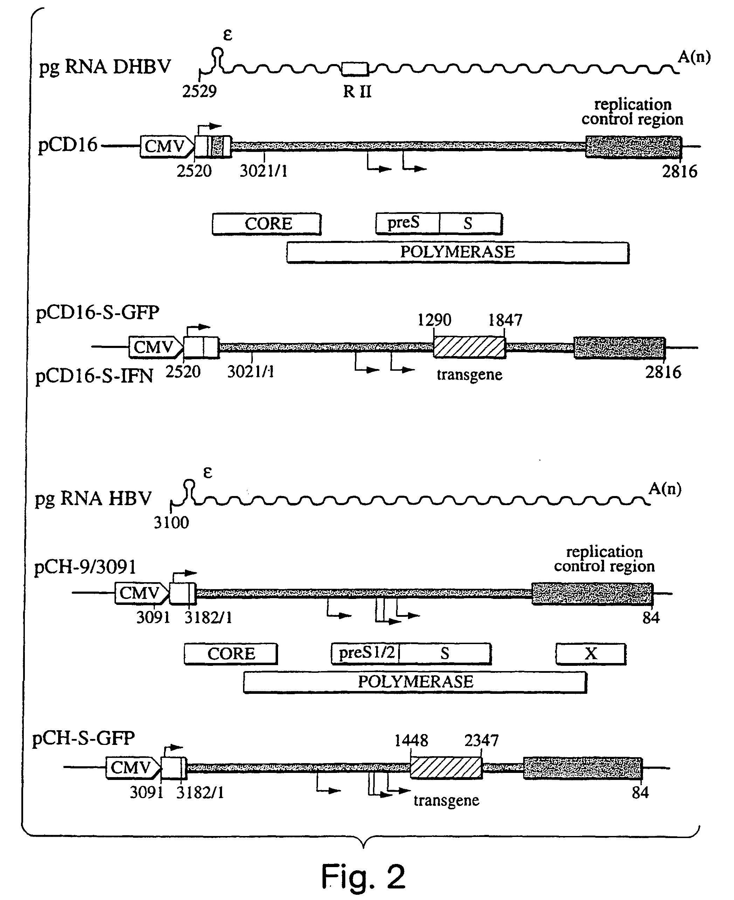

Figure 2 shows plasmid constructs used for the production of recombinant hepadnaviruses. The parental plasmids pCH-9/3091 (HBV) and pCD16 (DHBV) are based on terminally redundant hepadnavirus genomes (thick black lines) functionally mimicking the circular DNA genomes formed by reverse transcription of the RNA pregenomes (sinuous lines with A(n) representing the poly(A) tails). Numbers refer to nucleotide positions. The replication control regions (heavy black lines), encompassing HBV nucleotides 2360 to 40 and DHBV nucleotides 2100 to 2800, include *cis* signals for pregenomic RNA synthesis and maturation, and for RNA encapsidation and reverse transcription. These are continuous on the authentic circular viral genomes and partially duplicated here to create the terminal elements required for replication of the linearized genomes. Transcription start sites are indicated by the attached arrows, authentic viral genes by the open bars with the gene designations inside. The positions of the transgenes in the recombinant plasmids are shown by the hatched boxes. Synthesis of
the pregenomic RNAs is driven by a cytomegalovirus-IE enhancer/promoter element (marked CMV), whereas subgenomic RNAs, which encode the preS/S and the S envelope proteins and, for HBV, the X protein, are produced from internal promoters. In the RNA pregenomes, ε denotes a 5'-proximal stem-loop that, in the case of DHBV, acts together with a second region (box marked R II) as an encapsidation signal. The 5'-terminal part of ε (HBV up to nucleotide 3142, DHBV up to nucleotide 2579) is deleted in the helper constructs used to provide the missing gene products in trans. In pCH-S-GFP, a fragment encompassing the S gene was replaced by a DNA fragment encoding GFP fused to the first three amino acids of S. In pCD-16-S-GFP and pCD-16-S-IFN, DNA fragments encoding GFP and duck IFN, respectively, replace the KpnI to BstEII fragment encompassing the DHBV S gene.

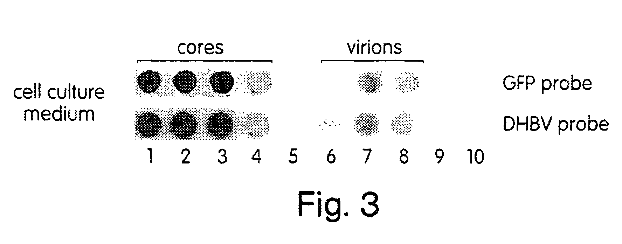

Figure 3 is a dot-blot analysis showing virion formation after cotransfection of DHBV transfer and helper plasmids into LMH cells. Cotransfection of the recombinant rDHBV-S-GFP plasmid, in which the S-gene is replaced by a GFP gene, with the helper plasmid which trans-complements the lacking viral envelope and P-proteins into LMH cells, resulted in virion formation. Enveloped virions were analyzed on a dot-blot membrane with DHBV- and GFP-specific probes.

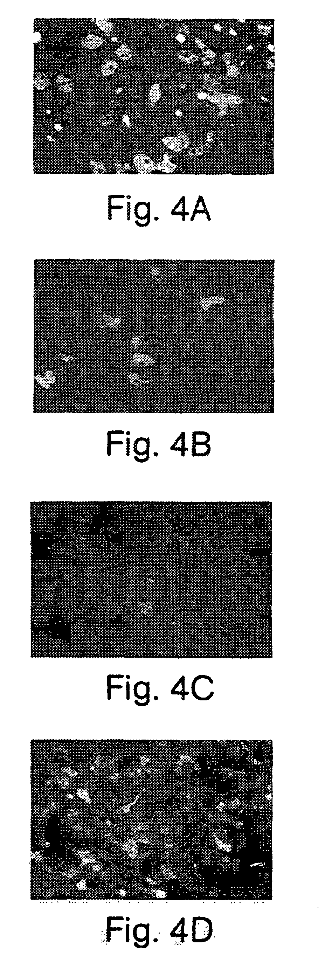

Figure 4A-D are photographs of hepatocytes depicting the transduction of primary hepatocytes by recombinant hepadnaviruses. Primary duck hepatocytes were infected at various multiplicities of infection with rDHBV-GFP, a recombinant DHBV that carries a GFP gene under the control of the DHBV S-promoter. GFP expression is shown (200-fold magnification) at day 6 post infection resulting from infection for 6 hours at a multiplicity of infection of 6 (Figure 4A), 25 (Figure 4B) or 100 (Figure 4C) or at a multiplicity of infection of 100 for 24 hours (Figure 4D).

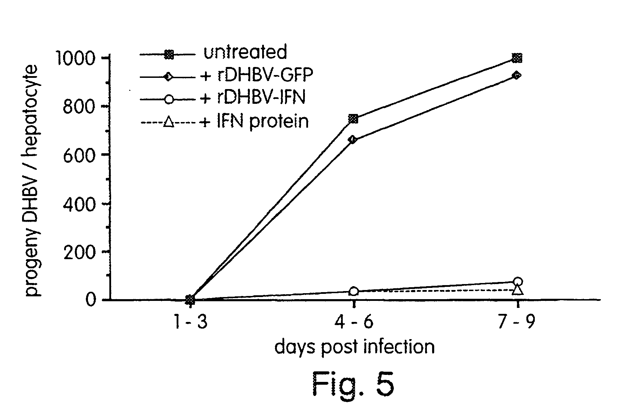

Figure 5 depicts the results from an experiment demonstrating that recombinant DHBV transferring an IFN gene interferes with the establishment of DHBV infection *in vivo*. Primary duck hepatocytes were infected with replication competent wildtype DHBV, and coinfected with recombinant DHBV which carried a gene coding for a duck homolog of alpha interferon (rDHBV-IFN) or with rDHBV-GFP as a negative control. Success of infection was monitored for release of progeny DHBV by DNA dot-blot and for structural DHBV proteins in cell lysates by Western blot. Figure 5 is a graph showing a quantitative evaluation of the time course of DHBV production (DHBV-DNA equivalents). Coinfection with rDHBV-IFN interfered with the establishment of a productive DHBV infection as effectively as interferon protein added at a dose showing maximal inhibition.

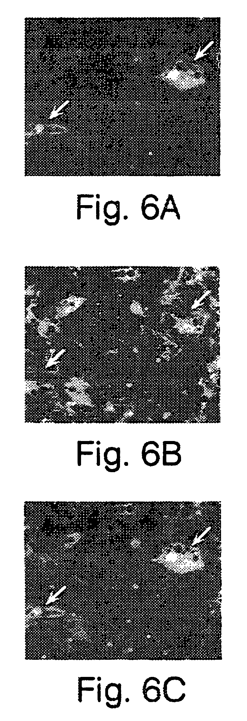

Figure 6A-C are photographs demonstrating that recombinant DHBV superinfects DHBV infected hepatocytes. Productively DHBV-infected hepatocytes were incubated with rDHBV-GFP (MOI of 50). After 6 days, cells were investigated for GFP fluorescence (Figure 6A), and stained for DHBV S-protein using a red-fluorescent TRITC-labeled secondary antibody (Figure 6B). As confirmed by the overlay (Figure 6C), GFP-expressing cells also stained positive for DHBV S-protein. Since the S-protein is expressed only from DHBV wildtype, but not from rDHBV-GFP, co-expression of GFP and S proves double-infection with both viruses.

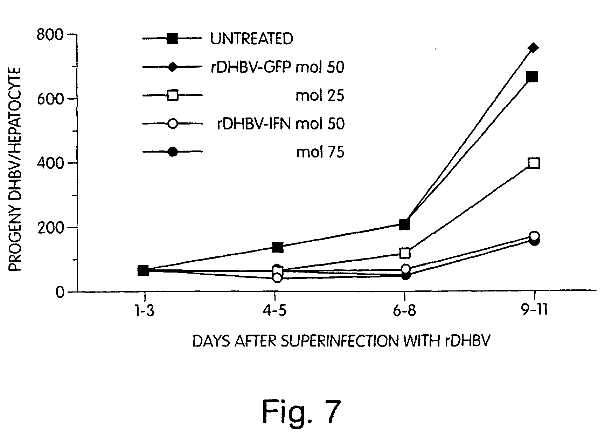

Figure 7 is a graph demonstrating therapeutic gene transfer by recombinant DHBV. DHBV preinfected hepatocytes were superinfected at various multiplicities of infection with rDHBV-IFN, or with rDHBV-GFP as a negative control. The time course of progeny DHBV release is shown.

### Detailed Description of the Invention

This invention pertains to methods and compositions relating to delivery of a foreign gene (*i.e.*, heterologous gene) into hepatocytes using a replication defective hepadnavirus particle. The invention is based, at least in part, on the discovery that replacement of nucleotide sequences in the S-gene region of hepadnavirus with a foreign gene, produce a replication defective hepadnavirus particle capable of infecting and expressing the foreign gene in hepatocytes at a level sufficient to interfere with the course of a viral infection in the hepatocytes. The data described herein demonstrate that the replication defective hepadnavirus particle acts as an effective delivery vector for a therapeutic gene to treat hepatic disorders.

The well-established duck hepatitis B virus (DHBV) model was used, which provides a readily available system for infection studies with primary hepatocytes as well as in whole animals, and efficacy in this model system is predictive of efficacy in human hepatitis B virus infection. Recombinant DHBV genomes were generated in which viral coding information was replaced by the green fluorescent protein (GFP) as a sensitive intracellular marker, or by a type 1 interferon (IFN) as a secreted protein with potential therapeutic applicability. Using these recombinant constructs, successful hepatocyte-specific transduction and expression of the transgenes *in vitro* and *in vivo* has been demonstrated herein (see the Examples).

Tissue-targeting, and in particular liver-targeting, is a major aim in gene therapy. Hepadnaviruses have distinct features that make them attractive candidates as vehicles for this purpose. In contrast to most retroviral vectors, they efficiently infect quiescent liver cells. Different from adeno-, herpes-, retro- and parvoviruses, virus uptake is hepatocyte specific, as is gene expression from hepadnaviral promoter/enhancer elements. Furthermore, hapadnaviruses encode only few gene products that might induce an anti-vector immune response, which is one of the major problems with adeno-and herpesvirus vectors. Finally hepadnaviral DNA does not obligatorily integrate into the host cell genome, which is especially important for transient expression of an effector gene as preferred for the treatment of acquired liver diseases.

The data disclosed herein provides strong experimental evidence for the practicability of hepadnavirus-based, liver-directed gene transfer system. They show for the first time: (1) that it is possible to generate, after replacement of viral coding information, high titers of recombinant virus particles carrying a functional transgene; (2) that these particles infect their target cells with the same high hepatocyte-specificity as the parental virus leading to strong expression of the foreign gene *in vitro* and *in vivo;* and (3) that transduction of an interferon gene blocks establishment of hepadnavirus infection and also substantially reduces virus production from preinfected hepatocytes. With titers above 10⁸ of enveloped virus particles per ml of cell culture supernatant and the possibility to further concentrate virus stocks without loss of infectivity, the recombinant hepadnaviruses described herein compare favorably with other vector systems such as retro- or parvoviruses.

Hepadnaviral gene transfer was found to be hepatocyte-specific; furthermore, all hepatocytes could be transduced *in vitro*. Although the transduction rate was reduced in the case of preinfected hepatocytes, a still significant fraction of productively DHBV-infected cells could be transduced by rDHV-GFP. These date were corroborated by the clear-cut inhibition of DHBV replication by rDHBV-IFN in co-infected, and, more importantly, also in DHBV preinfected liver cells. This latter fact indicates that there is no principal barrier to apply recombinant hepadnaviruses to the treatment of infectious liver diseases. Owing to their molecular properties, hepadnaviruses appear to be particularly suited for the transient liver-specific expression of foreign genes. Several important acquired liver diseases might be targeted by hepadnaviral vectors, including chronic infections by HBV or hepatitis C virus, which are among the most common and most severe viral infection of humans worldwide. Although the size of foreign DNA which can be integrated into recombinant hepadnaviruses is limited (e.g., to approximately 800 bp of inserted DNA, although additional DNA may be accepted when other nonessential viral DNA sequences are deleted elsewhere in the genome), numerous important effector genes fit into the restricted space on the hepadnaviral genome. These include genes coding for specific antisense-RNA or trans-dominant proteins, as well as most cytokine genes.

Systemic treatment with IFN alpha currently is the only approved therapy for chronic hepatitis B and C. Other cytokines such as IFN gamma or TNF alpha potently suppress liver infections with viral and non-viral agents, such as malaria hepatic stages, but severe side effects prohibit their systemic high-dose application. Therefore, local expression after liver-directed gene-transfer using the recombinant hepadnaviral vectors provided herein may provide a more efficient and better tolerated alternative.

In one aspect, the invention pertains to a method for expressing a heterologous gene in hepatocytes. The method involves:
providing replication defective hepadnavirus particles at a titre level competent to infect hepatocytes, wherein a region of at least about 200 nucleotides of the S-gene of the hepadnavirus genome has been replaced with the heterologous gene of about 200 up to about 1200 nucleotides such that expression of the heterologous gene is regulated by regulatory sequences of the S-gene; and
infecting hepatocytes with the hepadnavirus such that the heterologous gene is delivered into the hepatocytes and expressed in the hepatocytes.

Preferably, the replication defective hepadnavirus particles are human hepatitis B virus particles.

In one embodiment, the heterologous gene replaces the region of the S-gene such that nucleotides encoding at least one amino acid of the S protein are fused in-frame to the 5' end of the heterologous gene. In another embodiment, the heterologous gene replaces the sequence of the S-gene at a site equivalent to the KpnI site at position 1290 of duck hepadnavirus. In still another embodiment, the heterologous gene replaces a region of the S-gene at a site equivalent to the KpnI site at position 1290 of duck hepadnavirus, and the heterologous gene is inserted such that nucleotides encoding at least one amino acid of the S protein are fused in-frame to the 5' end of the heterologous gene. In still another embodiment, the heterologous gene replaces a region of the S-gene, and the heterologous gene is inserted such that nucleotides encoding at least one amino acid of the S protein are fused in-frame to the 5' end of the heterologous gene. In yet another embodiment, the heterologous gene replaces the S-gene. In still another embodiment, the heterologous gene replaces the S-gene and at least part of the preS-region.

Preferred heterologous genes for expression in hepatocytes are genes encoding modulating agents (i.e., agents that modulate a viral infection of the hepatocytes or other disorder of the hepatocytes). Preferred modulating agents are cytokines. A particularly preferred cytokine is IFNα (Type I IFN).

Another aspect of the invention pertains to a method of treating a subject with a hepatic disorder. The method involves:
providing replication defective hepadnavirus particles at a titre level competent to infect hepatocytes of the subject with the hepatic disorder, wherein a region of the S-gene of the hepadnavirus genome has been replaced with a therapeutic gene such that expression of the therapeutic gene is regulated by regulatory sequences of the preS/S-gene; and
infecting hepatocytes of the subject with the hepadnavirus particles such that the therapeutic gene is delivered into the hepatocytes and expressed in the hepatocytes at a level sufficient to treat the hepatic disorder.

Preferred hepatic disorders to be treated by the method include hepatitis B, hepatitis C, hepatocellular carcinoma, cirrhosis, steatosis, hemochromatosis, and inherited liver disorders.

Preferred therapeutic genes include genes encoding modulating agents, such as cytokines. Preferred cytokines include IFNα, IFNβ, IFNγ , EL-18 and TNFα.

In one embodiment, the hepadnavirus particle is directly administered to the subject. In another embodiment, the hepadnavirus construct and a helper virus construct are cultured *in vitro* and the infectious particles produced from the culture are administered to the subject. In another embodiment, recombinant hepadnavirus particles are produced by a helper cell line, and are administered to the subject.

The invention also provides a method of treating a subject with a hepatitis infection. The method involves:
providing replication defective hepadnavirus particles at a titre level competent to infect hepatocytes of the subject with hepatitis, wherein a region of the S-gene of the hepadnavirus genome has been replaced with a gene encoding a cytokine such that expression of the gene encoding a cytokine is regulated by regulatory sequences of the preS/S-gene; and
infecting hepatocytes of the subject with the hepadnavirus such that the gene encoding a cytokine is delivered to the hepatocytes and expressed in the hepatocytes at a level sufficient to treat the hepatitis.

Preferably, the cytokine is IFNα. Alternatively, the cytokine can be, for example, TNFα, IFNβ, IL-18 or IFNγ.

In a particularly preferred embodiment, the hepatitis infection is hepatitis B and the cytokine is IFNα.

Another aspect of the invention pertains to a replication defective hepadnavirus particle, wherein a region of at least about 200 nucleotides of the S-gene of the hepadnavirus genome has been replaced with a therapeutic gene (e.g., a cytokine gene, such as such INFα) of about 200 up to about 1200 nucleotides such that expression of the therapeutic gene is regulated by regulatory sequences of the S-gene. Pharmaceutical compositions comprising the replication defective hepadnavirus particle and a pharmaceutically acceptable carrier or the replication defective hepadnavirus particle and a helper virus are also encompassed by the invention.

Yet another aspect of the invention pertains to a method of producing therapeutic replication defective hepadnavirus particles at a titre level suitable for therapeutic use. The method involves:
co-transfecting a hepatoma cell line with:
   (i) replication defective hepadnavirus constructs, wherein a region of at least about 200 nucleotides of the S-gene of the hepadnavirus DNA has been replaced with a therapeutic gene of about 200 up to about 1200 nucleotides ,preferably a cytokin regulated by regulatory sequences of the S-gene; and
   (ii) a helper construct;
culturing these cell s until infectious viral particles are produced; and recovering the infectious viral particles.

So that the invention may be more readily understood, certain terms are first defined.

As used herein, the term "hepadnavirus" refers to a member of the *Hepadnaviridae* family of viruses, including but not limited to, human hepatitis B virus, wooly monkey hepatitis virus (Lanford *et al*. (1998) *Proc. Natl*. *Acad*. *Sci. U*.*S*.*A*. 95: 5757-5761), duck hepatitis B virus (DHBV; Mandart *et al*., (1984) *J*. *Virol*. 49: 782-792; Mason *et al*., (1978) *J*. *Virol*. 36: 829-836), heron hepatitis virus (Sprengel *et al*., (1988) *J*. *Virol*. 62: 3832-3839), woodchuck hepatitis virus (Summers *et al*., (1978) *Proc*. *Natl*. *Acad. Sci.* 75: 4533-4537), and ground squirrel hepatitis virus (Marion *et al*., (1980) *Proc. Natl*. *Acad*. *Sci.* 77: 2941-2945). Examples of other hepadnaviruses within the scope of the invention include, but are not limited to, HBV strains infecting various human organs, including hepatocytes, exocrine and endocrine cells, tubular epithelium of the kidney, spleen cells, leukocytes, lymphocytes, e.g., splenic, peripheral blood, B or T lymphocytes, and cells of the lymph nodes and pancreas (*see* e.g. Mason *et al*., (1989) *Hepalology*. 9: 635-645). The invention also applies to hepadnaviruses infecting non-human mammalian species, such as domesticated livestock or household pets.

As used herein, the term "heterologous gene" or "foreign gene" refers to any gene or DNA sequence that does not occur naturally in the hepadnavirus genome, and which is incorporated into the hepadnaviral genome. The term "heterologous gene" or "foreign gene" is also used to encompass a DNA molecule from an entirely different species, *e.g.*, a human DNA sequence, *e.g.*, the gene encoding human INFα which is incorporated into the hepadnaviral genome.

As used herein, the term "replication defective hepadnaviral particle" refers to a hepadnavirus with a packaging signal (ε), in which a portion of the hepadnavirus genome has been replaced by a heterologous gene. The heterologous gene replaces a portion of the hepadnavirus genome which encode protein products essential for replication, and thereby renders the hepadnavirus incapable of replicating. Replication by the replication defective hepadnaviral particle is permissible with the help of a "helper virus" which can produce protein products that the replication defective hepadnaviral particle is incapable of producing. In particular, the term "replication defective hepadnaviral particle" refers to a hepadnavirus in which at least a portion of the S-gene, which encodes envelope proteins, is replaced.

As used herein, the term a "region" or "portion" of a gene (e.g., the hepadnaviral S gene) refers to a length of nucleotide sequence of the hepadnavirus genome which is replaced by a heterologous gene. Preferably the length of replaced nucleotide sequence is at least about 200, preferably at least about 300 or 400, and even more preferably about 500 or 600 base pairs in length. Replacement of up to 800 nucleotides has been demonstrated.

As used herein, the term "regulatory sequences" is art-recognized and intended to include promoters, enhancers and other expression control elements (e.g., polyadenylation signals). Such regulatory sequences are known to those skilled in the art and are described in Goeddel, *Gene Expression Technology: Methods in Enzymology* 185, Academic Press, San Diego, CA (1990).

The term "titre level competent to infect" also refers to an amount (e.g., number of viral particles per a specified volume) sufficient to infect hepatocytes when applied to the hepatocytes. Suitable titre levels are for example, at least 3x10⁷/ml to 2x10⁸/ml of culture supernatant.

As used herein, the term "S-gene" refers to a hepadnaviral gene which encodes the S protein, a surface component of the hepadnavirus envelope (env). Expression of the S-gene is under the control of the SP2 promoter. Two additional surface proteins, which are also components of the envelope, are the Large (L) and Middle (M) proteins (these are derived from alternative start sites). The L protein is regulated by the SP1 promoter. The "preS" region encompasses the genetic region 5' of the S-gene, including the promoter and other transcriptional regulatory regions.

As used herein, the term "modulating agent" refers to a compound which alters the state of the hepatocyte, such as agents that alter or interfere with a viral infection of the hepatocytes or other disorder of the hepatocytes. Examples of modulating agents that can change the state of the hepatocyte include compounds which can eliminate or diminish a disease of the liver, for example, cytokines such as INFα, IFNβ, INFγ, TNF and IL-18. Other examples of modulating agents include those that alter the function of hepatocytes, for example, to improve enzyme metabolism.

As used herein, the term "treating" refers to a reduction, alleviation or amelioration of at least one adverse effect or symptom of a disease or disorder, e.g., a disease or disorder associated with hepatitis B virus infection, for example hepatitis B, cirrhosis or hepatocellular carcinoma.

As used herein, the term "subject" is intended to include organisms that are capable of being infected by hepadnaviruses, included mammals and birds. Preferred subjects are mammals. Examples of subjects include humans, ducks, woodchucks, squirrels, monkeys, dogs, cats, mice, rats cows, horses, goats, and sheep.

As used herein, the term "hepatic disorder" refers to any disease associated with the liver. Examples of diseases within the scope of the invention include, but are not limited to, hepatitis B, hepatitis C, hepatocellular carcinoma, cirrhosis, steatosis, hemochromatosis, and inherited liver disorders.

As used herein, the term "therapeutic gene" refers to a gene which encodes a therapeutic polypeptide which reduces; alleviates or ameliorates at least one adverse effect or symptom of a hepatic disease or disorder. Examples of therapeutic genes within the scope of the invention include, but are not limited to cytokines such as, INFα, IFNβ, INFγ, TNF, IL-18, antisense oligonucleotides, or inhibitory peptides.

As used herein, the term "helper construct" or "helper virus" refers to a virus which can produce protein products that the replication defective hepadnaviral particle is incapable of producing. The "helper virus" provides every factor essential for replication and renders the replication defective hepadnaviral particle capable of replicating. An Example of a helper construct within the scope of the invention includes, but is not limited to, a hepadnavirus construct lacking the envelope packaging signal (ε), and the hepatitis B virus. An example of a helper construct for HBV is pCH3142, and for DHBV is pCD4 (described further in the Examples).

The molecular biology of hepadnaviruses and their infectious cycle has been well characterized (for reviews see e.g., Nassal *et al*., (1993) *Trends Microbiol*. 1:221-228; Nassal, *et al*., (1996) *J. Viral Hepat*. 3:217-226). Infectious virions contain a partially double-stranded circular DNA genome of only 3 - 3.2 kb in length, with the viral replication enzyme, P protein, covalently attached to the 5'-end of the long DNA strand. After entry into the host cell, the genome is delivered to the nucleus and transformed into a covalently closed circular DNA (cccDNA) that serves as a template for transcription of four classes of subgenomic and genomic RNAs. All the RNAs are translated into protein; the mRNA for the capsid and the P protein is, in addition, copackaged with P protein into newly forming capsids where it is reverse transcribed by the enzyme into DNA. It is therefore termed RNA pregenome, or pregenomic RNA. A number of cis-elements have been identified which are required to ensure efficient production of genomic and subgenomic transcripts, and for packaging and reverse transcription of the pregenomic RNA. These include promoters and enhancers (Hirsch *et al*. (1991) *J*. *Virol*. 65:3309-3316; Schaller *et al*. (1991) *Curr*. *Topics Microbiol. Immunol*. 168:21-39), the poly-adenylation signal, the RNA encapsidation signal ε (Hirsch *et al*. (1991) *J*. *Virol*. 65:3309-3316; Junker Niepmann *et al*., (1990) *Embo J*. 9:3389-3396), a less well defined DHBV-specific second element, region II (Calvert *et al*., (1994) *J*. *Virol*. 68:2084-2090), and several copies of a direct repeat sequence (DR1, DR2 and DR1*) (Lien *et al*., (1987) *J*. *Virol*. 61:3832-3840; Molnar Kimber *et al*., (1984) *J. Virol.* 51:181-191; Seeger *et al*., (1991) *J. Virol*. 65:5190-5195). Further cis-elements, for example, the PET element (Huang, *et al*. (1994) *J*. *Virol*. 68:1564-1572), are involved in transcriptional regulation and intracellular transport.

Two major animal virus models are currently used as infection systems for HBV: the woodchuck hepatitis B virus (WHV; (Roggendorf *et al*., (1995) Intervirology 38:100-112)) and the duck hepatitis B virus (DHBV; (Schodel *et al*., (1991). In particular ducks provide a readily available system for infection studies in whole animals as well as in primary liver cells.

Gene delivery by a hepadnaviral vector requires generation of infectious, but preferentially replication defective recombinant virus particles. To generate infectious virus particles, recombinant pregenomic RNA must meet some requirements that limit the possibilities for inserting additional foreign sequences. The most important constraints are the small size and compact organization of the hepadnaviral genome that precludes simple insertion of additional sequences. An insertion may interfere with one or more of the numerous cis-elements that make up approximately 15% of the viral genome. Thus, replacement of coding information by a foreign gene may be suitable to generate a replication deficient recombinant virus.

In one aspect, the invention pertains to a method for expressing a heterologous gene in hepatocytes by providing replication defective hepadnavirus particles at a titre level competent to infect hepatocytes, wherein a region of the S-gene of the hepadnavirus genome has been replaced with the heterologous gene such that expression of the heterologous gene is regulated by regulatory sequences of the S-gene, and infecting hepatocytes with the hepadnavirus such that the heterologous gene is delivered into the hepatocytes and expressed in the hepatocytes.

The molecular biology of hepadnaviruses and their infectious cycle has been well characterized (for reviews see e.g., Nassal *et al*., (1993) *Trends Microbiol*. 1:221-228; Nassal, *et al*., (1996) *J*. *Viral Hepat*. 3:217-226). Infectious virions contain a partially double-stranded circular DNA genome of only 3 - 3.2 kb in length, with the viral replication enzyme, P protein, covalently attached to the 5'-end of the long DNA strand. After entry into the host cell, the genome is delivered to the nucleus and transformed into a covalently closed circular DNA (cccDNA) that serves as a template for transcription of four classes of subgenomic and genomic RNAs.

To generate infectious virus particles, recombinant pregenomic RNA must meet some requirements that limit the possibilities for inserting additional heterologous sequences. The most important constraints are the small size and compact organization of the hepadnaviral genome that precludes simple insertion of additional sequences. An insertion may interfere with one or more of the numerous cis-elements that make up approximately 15% of the viral genome.

Many strategies are known in the art to produce constructs of the hepadnavirus gene. The relevant sequences of the hepadnaviral genome and of the heterologous gene can be cleaved at appropriate sites with restriction endonucleases, isolated and ligated in vitro, using techniques known in the art. In the method of the invention, a region of the S-gene of the hepadnavirus gene is replaced with the heterologous gene. In preferred embodiments, the heterologous gene is inserted into a region of the preS/S-gene. In other preferred embodiments, the heterologous gene is inserted into a region of the preS/S-gene such that nucleotides encoding at least one amino acid of the S protein are fused in-frame to the 5' end of the heterologous gene. Preferably, the heterologous gene is operably linked with least one amino acid of the S protein. More preferably, the heterologous gene is operably linked with up to five to ten amino acids of the S protein. More preferably, the heterologous gene is operably linked with one, two, three amino acids of the S protein. Most preferably, the heterologous gene is operably linked with four amino acids of the S protein. Example 1 shows the construct which demonstrates the efficiency of operably linking four amino acids of the S protein with green fluorescent protein (GFP). When the construct was transfected into chicken hepatoma cell line (LMH) cells a bright green fluorescence was detected 48 hours post transfection, demonstrating efficient expression of GFP.

In preferred embodiments, the heterologous gene replaces a region of the S-gene at a site equivalent to the KpnI site at position 1290 of duck hepadnavirus. In other preferred embodiments, the heterologous gene is inserted into the preS/S-gene after the authentic AUG. Accordingly, the nucleotide sequence of any hepatitis virus, such as human hepatitis B virus may be used and the equivalent KpnI site used to clone the heterologous gene. Alternatively, the nucleotide sequence of any hepatitis virus, such as human hepatitis B virus may be used and the heterologous gene inserted after the authentic AUG in either the preS/S-gene.

The size of the heterologous gene used to replace the S-region is preferably about 200 up to about 1200 nucleotides. More preferably, the size of the heterologous gene used to replace the S-region is about 300 up to 800 nucleotides. Most preferably, the size of the heterologous gene used to replace the S-region is about 600 nucleotides.

In preferred embodiments, the heterologous gene encodes a modulating agent which modulates the state of the liver once the replication defective hepadnavirus particles containing the heterologous gene is expressed in the liver. Examples of modulating agents include compounds that can change the state of the liver include those which can eliminate, ameliorate or improve a disease of the liver. Other examples of modulation include those that alter the function of hepatocytes, for example, to improve enzyme metabolism. Modulating agents include, but are not limited to cytokines, blood factors, enzymes, antisense nucleic acids, and transdominant proteins. In preferred embodiments, the modulating agent is a cytokine. In still more preferred embodiments, the cytokine is INFα.

In another aspect the invention provides a method of treating a subject with a hepatic disorder by providing replication defective hepadnavirus particles at a titre level competent to infect hepatocytes of the subject with the hepatic disorder, wherein a region of the S-gene of the hepadnavirus genome has been replaced with a therapeutic gene such that expression of the therapeutic gene is regulated by regulatory sequences of the preS/S-gene; and infecting hepatocytes of the subject with the hepadnavirus particles such that the therapeutic gene is delivered into the hepatocytes and expressed in the hepatocytes at a level sufficient to treat the hepatic disorder. As demonstrated in the examples, intravenous injection of infectious replication defective hepadnavirus particles is sufficient to deliver the particles into hepatocytes *in vivo* and to achieve expression of the heterologous gene in the hepatocytes.

Hepatic disorder that can be modified by modulating agents include transient hepatic disorder which require treatment with the replication defective hepadnavirus particles wherein a region of the S-gene of the hepadnavirus genome has been replaced with a therapeutic gene only until the hepatic disorder has been ameliorated. Examples of transient hepatic disorders include, but are not limiting to hepatitis B, hepatitis C, cirrhosis, hepatocellular carcinoma, and malaria. Other hepatic disorders that can be treated using the method of the invention include, but are not limited to hyperammonnemia, infantile cholestansis and hematomegaly.

In order to directly demonstrate the potential of hepadnaviral vectors for liver-specific gene delivery, recombinant DHBV and HBV particles carrying a foreign gene were generated, and used to infect primary duck or human hepatocytes. Using the green fluorescent protein (GFP) as a marker, several recombinant DHBV and HBV genomes were constructed, some of which yielded substantial titers of secreted recombinant virus (rDHBV or rHBV). These viruses infected primary duck or human hepatocytes in a species-specific manner and efficiently delivered the foreign gene as demonstrated by GFP fluorescence. As a candidate therapeutic agent a homologous recombinant DHBV carrying the duck type I interferon (DuIFN) (Schultz *et al*., (1995) *Virology* 212:641-649) was generated. Infection of primary hepatocytes from endogenously infected ducks with this recombinant reduced DHBV replication by more than 90%.

### Examples

The present invention is further illustrated by the following examples.

Methodologies and reagents utilized in the following Examples are described below:

### I. DHBV Methodologies

DHBV Plasmid constructs. All DHBV constructs are based on plasmid pCD 16 which contains a terminally redundant DHBV genome (subtype 16) (Mandart, E. et al. (1984) *J. Virol*. 49:782-792) (3317 bp, nucleotides 2520 to 2816) under control of the CMV immediate early promoter/enhancer (Fig. 1A-B and 2)(Obert, S. et al. (1996) *EMBO J.* 15:2565-2574; Bartenschlager, R. (1990) Thesis, University of Heidelberg). The helper plasmid pCD4 lacks part of the 5 encapsidation signal ε (Fig. 1A-B and 2) and is therefore encapsidation-deficient (Bartenschlager, R. (1990) Thesis, University of Heidelberg). The marker construct pCD16-S-GFP was obtained by replacing a DHBV-DNA fragment containing the S gene (from Kpn I, nucleotide position 129D, to BstE II, nucleotide position 1847; see Fig. 1A-B and 2) with a PCR fragment (733 nucleotides) encoding a fluorescence-enhanced, red-shifted GFP prepared from plasmid pTR-UF5 (Zolotukhin, S., et al. (1998) *J*. *Virol*. 70:4646-4654) with primers introducing terminal Kpn I and BstE II sites. pCD16-S-IFN was obtained analogously by inserting a PCR-derived fragment (591 nucleotides) encoding the complete duck type 1 IFN gene (Schultz, U. et al. (1995) *J*. *Virol*. 70:4646-4654). For production of recombinant duck IFN protein, the IFN gene was cloned into a pUC based CMV-IE promoter controlled expression vector (pCD IFN).

Production of recombinant DHBV stocks. Chicken hepatoma LMH cells (Condreay, L.D. et al. (1990) *J*. *Virol*. 64:3249-3258) at 30-40% confluency were cotransfected using the calcium-phosphate method with 50µg of the respective recombinant pCD16 plus 25µg plasmid pCD4 per 15 cm dish. Cell culture medium containing recombinant virions was collected from days 3 to 5 post transfection, concentrated 10- to 50-fold by precipitation with 6.5% polyethylene glycol 20.000/0.9% NaCl at 0°C, and stored in PBS/10% glycerol at -20°C until further use. Virus titers, measured as enveloped DNA containing particles, were determined by density gradient centrifugation and subsequent dot blot analysis relative to a DHBV-DNA standard (Obert, S. et al. (1996) *EMBO J.* 15:2565-2574).

Isolation of primary hepatocytes for DHBV experiments. Primary duck hepatocytes (PDH) were isolated from 2- to 3-week-old Peking ducks by a standard two step collagenases perfusion via the portal vein and subsequent differential centrifugation (50 x g), seeded at a density of 2.5 x 10⁵ cells/cm²) and maintained as described (Hild, M. et al. (1998) *J*. *Virol*. 72:2600-2606). DHBV-positive PDH were obtained analogously from ducks experimentally infected the first day after hatching with 100 µl duck serum containing 10⁹ DHBV16-virions. Primary mouse hepatocytes were isolated from 16 to 20-week old CH57BL/6 mice, seeded onto collagen type 1 (Sigma Aldrich, Irvine, CA, USA) coated tissue culture plates in maintenance medium/10% FCS at 4 x 10⁵ cells/cm² and maintained without FCS as described (Hild, M. et al. (1998) *J*. *Virol*. 72:2600-2606). Endothelial cells and Kupffer cells in the hepatocyte cultures were identified on the basis of their uptake of a TRITC labeled acetylated low-density lipoprotein (Dil-Ac-LDL; Paesel & Lorel, Duisburg, Germany (Irving, M.G. et al. (1984) *Gastroenterology* 87:1233-1247)) and by phagocytosis of 1µm amine-modified yellow-green fluorescent latex beads (Sigma-Aldrich Chemie, Deisenhofen, Germany), respectively.

DHBV infection and gene transfer by recombinant DHBV. Primary hepatocytes were incubated, at day 2 post plating, for 24 hours with rDHBV, or wildtype DHBV from a DHBV15 positive duck serum, diluted in maintenance medium at the desired multiplicity of infection. GFP expression was monitored by fluorescence microscopy using a standard FITC-filter set with excitation by blue light (488 nm). For *in vivo* infections ducklings were inoculated at day one after hatching with 10⁹ rDHBV-GFP virions. At day 7 post-infection, animals were anaesthetized and perfused via the portal vein with cold 4% paraformaledhyde/0.25% glutaraldehyde. Livers were removed, post-fixed for 24 hours in perfusion buffer, saturated with 30% sucrose and sectioned serially (10 - 15µm) on a freezing microtome. In addition, primary hepatocytes were isolated and analyzed as described above.

Coinfection of DHBV-positive pDH with recombinant DHBV-IFN.
DHBV-negative PDH were simultaneously infected with serum-derived DHBV (multiplicity of infection of 25) plus rDHBV-IFN (multiplicity of infection of 50) or plus rDHBV-GFP (multiplicity of infection of 50). DHBV-positive PDH were infected accordingly. Cell lysates were analyzed for Intracellular DHBV proteins by Western blot analysis (described below), and release of progeny DHBV virus into the cell culture medium was quantitatively determined by DHBV-DNA dot blot analysis. As a positive control, DHBV-infected PDH were incubated with a diluted preparation of recombinant duck IFN protein obtained in the form of cell culture medium of LMH cells transfected with plasmid pCD-IFN at a dose which had proven in previous experiments sufficient to maximally inhibit DHBV replication. IFN protein was added at day 3 post infection at which time transgene expression from rDHBV-GFP was first detectable.

Immunofluorescence staining and Western blot analysis of intracellular DHBV antigens. For immunodetection of intracellular DHBV antigens a polyclonal rabbit antiserum against the DHBV core-protein (Schlichl, H.J. et al. (1987) *J*. *Virol*. 61:3701-3709), or monoclonal antibody MAb 7C.12 (Pugh, J.C. et al. (1995) *J*. *Virol*. *59*:4814-4822) recognizing the DHBV S-protein were used and detected with an appropriate fluorescence-labeled secondary antibody. For direct detection of intracellular viral proteins, 10⁶ PDH were lysed by the addition of 250 µl of protein sample buffer (Hild, M. et al. (1998) *J*. *Virol*. 72:2600-2606) after removal of cell culture medium. In addition, cytoplasmic lysates from 10⁷ transfected LMH cells were incubated with rabbit antiserum against DHBV pre S-protein (Schöcht, H.J. et al. (1987) *J .Virol*. 61:2280-2285) or with rabbit antiserum against GFP (Clontech, Palo Alto, CA, USA) antibodies and immunoprecipitated proteins were released by boiling the beads in 50 µl sample buffer (Schöcht, H.J. et al., *supra*). 25 µl each were separated by 10% SDS-PAGE, blotted to a positively charged nylon membrane, immunostained with the polyclonal antisera against DHBV core- (Schlichl, H.J. et al., *supra*), S- (Schöcht, H.J. et al., *supra*) and pre S-proteins (Schlichl, H.J. et al., *supra*) or against GFP, and visualized using the ECL-system (Amersham, Cleveland, Ohio, USA) essentially as described (Hild, M. et al. (1998) *J*. *Virol*. 72:2600-2606).

### II. HBV Methodologies

HBV Plasmid constructs. HBV constructs contained under control of the CMV immediate early promoter/enhancer a terminally redundant genome of HBV, subtype ayw 1 (pCH-9/3091, HBV nucleotides 3091 to 84, numbering from the core initiation codon) (Nassal, M., et al. (1990) *Cell* 63: 1357-1363). The helper construct pCH3142 (Bartenschlager, R. (1990) Thesis, University of Heidelberg) lacked part of the 5' encapsidation signal ε and is therefore encapsidation-deficient (Fig. 2).

The marker construct pCH-S-GFP was obtained by replacing DNA fragments containing the S gene (from XhoI, nucleotide position 1409, to NsiI, nucleotide position 2347) with a PCR fragment (733 nucleotides) encoding a fluorescence-enhanced, red-shifted GFP prepared from plasmid pTR-UF5 (Zolotukhin, S. et al. (1996) *J*. *Virol*. 70: 4646-4654) with expression being expected to be driven by the S promoter (see Fig. 2).

Production of recombinant HBV stocks. Human hepatoma HuH7 cells (Chang, C.M. et al. *EMBO J*. 6: 675-680) were cotransfected with rHBV and helper construct using the same methodologies employed in the production of recombinant DHBV stocks, above. Wildtype HBV was produced by transfecting HuH7 cells with plasmid pCH-9/3091.

Isolation of primary hepatocytes for HBV experiments. Surgical human liver biopsies were obtained after informed consent of the donor and perfused via a large branch of the portal vein after disclosure of small vessels. Primary hepatocytes were isolated by a standard two step collagenase perfusion and subsequent differential centrifugation (at 50 x g), as described above in the procedures for the isolation of primary hepatocytes for DHBV experiments.

HBV infection and gene transfer by recombinant HBV. Primary human hepatocytes were incubated for 24 hours with rHBV-S-GFP or wildtype HBV, diluted in maintenance medium at a multiplicity of infection of 500, at day 1 post plating. GFP expression was monitored as described in the DHBV infection experiments (above).

Immunofluorescence staining and Western blot analysis of intracellular HBV antigens For immunodetection of intracellular HBV antigens, polyclonal rabbit antisera against the HBV core-protein were used and detected with an appropriate fluorescence-labeled secondary antibody. For direct detection of intracellular HBV proteins, infected human hepatocytes were lysed and subjected to Western analysis under similar conditions to those utilized for the DBHV-infected PDH cells (above).

### Example 1: Preparation of Duck Hepatitis B Virus Plasmid Constructs

Plasmid constructs used for transfection into cells were prepared from the parental plasmid, pCD16, which contains an overlength DHBV 16 genome (nucleotide 2520 to 3021/1 to 2816) under control of the CMV immediate early promoter (Bartenschlager, R. et al. (1990) *J*. *Virol*. 64:5324-5332). Upon transfection of the plasmid into the chicken hepatoma cell line LMH (Condreay *et al*., (1990) *J. Virol*. 64:3249-3258), genomic transcripts starting at position 2529 and terminating around nucleotide 2800 (Figure 1A) are produced that give rise to the formation of infectious DHBV particles (Obert *et al*., (1996) *EMBO J*. 15:2565-2574). pCD4 (Bartenschlager, R. et al. (1990) *J*. *Virol.* 64:5324-5332) is a derivative of pCD16 containing an overlength DHBV genome (nucleotides 2589 to 2845) lacking the 5' encapsidation signal Dε (Figure 1B); it provides all gene products in *trans* but is itself encapsidation, and therefore, replication-deficient (analogous HBV constructs have been described (Junker Niepmann *et al*., (1990) *EMBO J*. 9:3389-3396).

As a marker, a fluorescence-enhanced variant (S65T/F64L, humanized codon usage) of the green fluorescent protein (GFP) present in plasmid pTR-UF5 (Zolotukhin *et al*., (1996) *J .Virol*. 70:4646-4654), was used. Using appropriate primers, the GFP gene was modified by PCR to carry additional terminal restriction sites. These allowed cloning of the GFP gene between the Kpn I (nucleotide position 1290) and BstE II (nucleotide position 1847) sites in plasmid pCD16, thus replacing the S-gene (Figure 1B). With 3196 base pair (bp), the resulting rDHBV-S-GFP genome is 175 bp longer than authentic DHBV (3021 bp). Alternatively, the core gene fragments Xba I (nucleotide position 2662) to Hinc II (nucleotide position 141), or Xba I (nucleotide position 2662) to Bgl II (nucleotide position 391) were replaced; this left the De signal and the PET element intact. To provide a functional poly-adenylation signal, the canonical AAUAAA motif and following GU-rich sequence were also maintained. The foreign protein encoded by the resulting rDHBV-core-GFP genomes of 3299 bp and 3049 bp is a fusion of GFP to the N-terminal core protein amino acids 1 to 5 and 39 to 56.

In the plasmid construct, rDHBV-S-GFP, the GFP gene replaces essentially the entire S-gene except for its first four codons. Transcription of a subgenomic mRNA from the recombinant DHBV genome as well as from the pCD16-S-GFP expression construct occurs from the S and, possibly, the preS promoter (Buscher *et al*., (1985) *Cell* 40:717-724); in the latter case a preS/GFP fusion might be produced. For the core replacement constructs, a GFP encoding, in this case, genomic mRNA is produced from the strong CMV-IE promoter in the pCD16-core-GFP plasmid, and from the genomic promoter after recombinant virus formation. For the sake of preserving important *cis*-elements in the 5'-part of the pregenome, the core replacement constructs encode an N-terminal fusion of GFP to amino acids 1 to 5 and 39 to 56 of the DHBV core protein.

To test for sufficient expression of functional GFP, the pCD16-S-GFP and pCD16-core-GFP constructs were transfected into LMH cells and monitored for GFP fluorescence. This resulted in bright green fluorescence easily detectable 24 hours post transfection with the core-replacement constructs, and 48 hours post transfection with the S-replacement constructs. The result demonstrated that functional mRNAs and GFP proteins were produced from the CMV-IE promoter (pCD16-core-GFP) as well as from the endogenous preS/S promoters (pCD16-S-GFP). GFP-specific Western blot analysis of extracts from pCD16-S-GFP transfected cells showed two closely spaced bands of approximately 30 kDa. Most probably these represent GFP protein and a fusion of GFP to the first 4 amino acids of the S open reading frame arising from translation initiation at its authentic AUG codon. No larger products representing a putative preS-GFP fusion protein could be detected.

As a gene of potential therapeutic use, PCR-derived fragments encoding the complete duck Type I IFN gene (DuIFN) (Schultz *et al*., (1995) *Virology* 212:641-649) were introduced into the same locations as the S-gene or the core gene in the DHBV plasmid. The corresponding recombinant genomes are 3055 bp (rDHBV-S-IFN), and 3158 bp or 2908 bp (rDHBV-core-IFN) in length.

To produce recombinant DuIFN, the complete DuIFN gene was cloned into an eukaryotic expression vector under the control of a CMV-IE promoter.

Additional description of the construction and production of recombinant DHBV is as follows:

As a basis for constructing recombinant DHBV (rDHBV) genomes, the plasmid pCD16 was used, which upon transfection gives rise to the production of infectious DHBV particles (Obert, S. et al. (1996) *EMBO J.* 15:2565-2574)(see Fig. 2). Care was taken not to affect parts of the DHBV genome harboring cis-acting control elements, such as the well characterized replication control region, which directs synthesis, packaging, and reverse transcription of the RNA pregenome (Seeger, C. & Hu, J. (1997) *Trends in Microbiol*. 5:447-450; Nassal, M. & Schaller, H. (1996) *J*. *Viral*. *Hepat*. 3:217-226; Ganem, D., Hepadnaviridae: The Viruses and Their Replication, in *Fields Virology* (eds. Fields, B.N., Knipe, D.M & Howley, P.M), pp. 2703-2737 (Lippincott-Raven Publishers, Philadelphia, 1996)) and several less well defined control elements primarily involved in RNA maturation (Obert, S., *supra*; Huang, J. & Liang, T.J. (1993) *Mol. Cell. Biol*. 13:7476-7486; Huang, M. & Summers, J. (1994) *J*. *Virol*. 68:1564-1572; Clavert, J. & Summers, J. (1994) *J*. *Virol*. 65:2084-2090) (see Fig. 2). Initial studies indicated that replacement of the small envelope (S) gene by foreign sequences, and maintaining genome size with respect to the wild type, was the most successful approach. Two analogous C gene replacement constructs failed to produce enveloped DHBV particles, although their overall genome size was within the same limits and no known cis-elements were affected. In the constructs selected for further use, appropriately end-modified fragments encoding GFP (Zolotukhin, S. et al. (1998) *J*. *Virol*. 70:4646-4654)(pCD16-S-GFP) and duck IFN type 1 (Schultz, U. et al. (1995) *Virology* 212:641-649)(pCD16-S-IFN) were fused to the first four codons of the DHBV S-gene with expression being expected to occur from the preceding DHBV S promoter (Fig. 2).

Upon transfection of plasmid pCD16-S-GFP a bright GFP fluorescence became detectable about 48 hours post transfection. GFP-specific Western blot analysis of extracts from pCD16-S-GFP transfected LMH cells showed two closely spaced bands of approximately 30 kDa, probably representing GFP and a DHBV-S/GFP-fusion. An additional RNA, initiating from the pre-S promoter, might serve for the expression of a preS/GFP fusion protein. However, no larger products corresponding to such a fusion protein were detected.

S gene replacement destroys the S, L and polymerase open reading frames. For production of recombinant virus in transfected chicken hepatoma cells (Condreay *et al*., (1990) *J. Virol*. 64:3249-3258), the corresponding proteins were transcomplemented by the cotransfected encapsidation deficient helper pCD4 (Schlicht *et al*., (1989) *Cell* 56:85-92; Bartenschlager, R. et al. (1990) *J*. *Virol*. 64:5324-5332). This resulted in the production of enveloped recombinant DHBV (rDHBV) as verified by CsCl density gradient centrifugation (Obert *et al*., (1996) *EMBO J.* 15:2565-2574). Virus titers of rDHBV-GFP and rDHBV-IFN varied between 3x10⁷ and 2.5x10⁶/ml in different experiments, comparable to those of wild-type DHBV produced by transfection of LMH cells (Obert *et al*., (1996) *EMBO J*. 15:2565-2574). Virus could be concentrated by polyethylene glycol precipitation up to 50-fold without loss. These data proved that enveloped, recombinant virions with replacement of viral sequences by foreign genes could efficiently be produced.

### Example 2: Production of Recombinant Duck Hepatitis B Virus Stocks

High titres of recombinant DHBV viral stocks were produced by transfection of recombinant pCD16 plasmids into LMH cells. Since the S and core gene replacements destroy essential viral genes, to transcomplement the according gene products of the DHBV transfer plasmids (Horwich *et al*., (1990) *J*. *Virol*. 64:642-650; Schlicht *et al*., (1989) *Cell* 56:85-92) the encapsidation deficient helper plasmid pCD4 (Figure 1B), in which part of the 5'-terminal Dε signal is deleted, was also used. Confluent LMH cells were split 1:8 the day before transfection in order to reach 30-40% confluency at transfection. 50 µg of the respective recombinant pCD16 plasmid and 25 µg of the helper plasmid pCD4 to transcomplement lacking DHBV proteins were cotransfected per 15 cm dish, using the standard calcium-phosphate method. DNA-precipitates were washed off after overnight incubation (day 1 post transfection) and cell culture medium was exchanged again the next day (day 2). Cell culture medium containing recombinant DHBV virions was harvested at day 5 and day 8 post transfection. Virus stocks were concentrated by precipitation with polyethylene glycol 20.000 (final concentration 6.5%) at 0°C and stored in PBS /10% glycerol at -20°C until further use. Repeated freezing and thawing was avoided.

Cotransfection of the pCD16-core-GFP constructs and helper plasmid pCD4 into LMH cells resulted in bright green fluorescence that was easily detectable 24 hours post transfection. With pCD16-S-GFP and helper plasmid pCD4 constructs, development of strong fluorescence took about 48 hours.

Formation of recombinant virions was identified by sedimentation in a CsCl gradient which separates naked DNA-containing DHBV core particles from enveloped virions. 2 ml aliquots of cell culture media or 200 µl aliquots of concentrated virus stocks diluted in 1.8 ml PBS were layered on top of CsCl step gradients (bottom to top: 0.5 ml each of CsCl density 1.4, 1.3 and 1.2 and 20 % sucrose in H₂O) and ultracentrifuged (3.5 h at 4°C, 58000 r.p.m. in an SW60 rotor) to separate enveloped virus particles from naked cores (Obert *et al*., (1996) *EMBO J.* 15:2565-2574). Twelve 180 µl fractions (bottom to top) were collected and DNA was detected by dot blot hybridization with a ³²P labeled full length DHBV- or GFP-probe (specific activity ∼10⁸ c.p.m./ µg).

Cell culture medium harvested at day 5 and at day 8, subjected to sedimentation in a CsCl step gradient, separated naked DNA-containing DHBV core particles (fractions 1 to 4, bottom to top) from enveloped virions (fractions 6 to 8) as shown in Figure 3 (Obert *et al*., (1996) *EMBO J*. 15:2565-2574). Individual fractions were then analyzed by DNA dot blot using DHBV and GFP specific probes. For rDHBV-S-GFP, DNA hybridizing to both the DHBV and the GFP probe was clearly detectable in fractions characteristic for enveloped virions (Figure 3), indicating that recombinant virions had been generated. By contrast, both core GFP constructs failed to produce clearly detectable signals in the corresponding fractions, and hence were not used in further experiments.

Virus titres, measured as enveloped DNA containing particles, were determined by quantitative comparison with a dilution series of a DHBV-DNA standard on the same blot using a phosphorimager (Molecular Dynamics, Sunnyvale, Ca., USA). For the rDHBV-S-GFP recombinant virus, the titer of DNA containing enveloped particles was determined by quantification of the signal intensities relative to a dilution series of pCD16 DNA standard on the same blot and found to be between 3x10⁷ and 2x10⁸ ml in different experiments. Thus, the titres of recombinant viruses achieved by transcomplementation are comparable to those of wildtype virus from transfected LMH cells (Obert *et al*., *supra*). Further concentration of virus stocks could easily be achieved by polyethylene glycol precipitation. These experiments demonstrated that replacing a 550 bp DHBV fragment with a foreign gene of about 750 bp did not interfere with enveloped particle formation. The negative results with the core-GFP constructs, on the other hand, emphasize the importance of the appropriate location for the gene replacement.

### Example 3: Isolation of Primary Duck Hepatocytes

Primary duck hepatocytes (pDH) were isolated by standard methods. Briefly, livers from two to four week old ducklings were perfused by two step collagenase perfusion technique via the portal vein, hepatocytes were sedimented three times at 50 g and seeded at a density of 10⁶ cells per ml (2.5x10⁵/ cm²) in 6- or 12-well plates essentially as described before (Galle *et al*., (1989) *Hepatology* 10:459-465). Cells were maintained at 37°C, 5% CO₂ in supplemented Williams E medium (50 µg/ml gentamycin, 50 µg/ml streptomycin, 50 IU/ml penicillin, 2.25 mM L-glutamine, 0.06% glucose, 23 mM HEPES- pH7.4, 4.8 µg/ml hydrocortisone, 1 µg/ml inosine, 1.5% DMSO). DHBV positive pDH were obtained from ducklings infected with 100 µl DHBV16 positive duck serum (10¹⁰ DNA genome equivalents / ml) the first day after hatching of which serum samples obtained at day 7 and day 14 proved DHBV-positive by DNA dot blot analysis.

Non-parenchymal liver cells (especially Kupffer cells and sinusoidal endothelial cells) have been reported to make up 3-20% of all cells in culture after collagenase perfusion and differential sedimentation of hepatocytes (Johnston *et al*., (1994) *Hepatology* 20:436-444). Endothelial cells and some Kupffer cells in the pDH cultures were identified on the basis of their receptor-mediated uptake of Dil-Ac-LDL (Paesel & Lorei, Duisburg, Germany (Irving *et al*., (1984) *Gastroenterology* 87:1233-1247) after an incubation for 1-2 hours, that is, acetylated low-density lipoprotein labeled with TRITC as a fluorescent dye.

According to the protocol described, primary hepatocytes were isolated from 16 to 20-week old CH57BU6 mice, seeded onto collagen type I (Sigma Aldrich, Irvine, Ca., USA) coated tissue culture plates in maintenance medium /10% FCS at a density of 4 - 5 x 10⁵ cells / ml (10⁵/ cm²) and maintained as described above.

### Example 4: Infection of Isolated Primary Duck Hepatocytes with Recombinant Duck Hepatitis B Virus Particles

To examine the infectivity of recombinant virions, primary duck hepatocytes were infected with rDHBV during the first days in culture (usually day 2 post plating). rDHBV was diluted in maintenance medium to the desired multiplicity of infection (measured as DNA-containing enveloped DHBV particles / cell) and incubated on pDH for 24 hours. As an infection control, duck serum containing 10¹⁰ / ml DNA genome equivalents was obtained from a 4- week old DHBV positive duck infected with a standard DHBV 16 stock. F or wildtype DHBV, successful infection was determined by immunofluorescence staining of intracellular viral antigens with polyclonal rabbit antisera to DHBV proteins (D084 recognizing the preS-domain of DHBV L-protein or D087 recognizing denatured DHBV core protein) and a DTAF-labeled secondary goat-anti-rabbit antibody. In addition, cell culture medium was checked for progeny virus by DNA dot-blot analysis as described above.

After infection with the DHBV-GFP plasmids, cultured cells were monitored daily for green fluorescence by fluorescence microscopy using a standard FITC-filter set with excitation by blue light (488 nm). GFP expression was also checked by Western blot analysis. At the time of strongly positive green fluorescence, 10⁶ primary hepatocytes were lysed in 250µl protein-sample buffer (200 mM Tris-HCl pH 8.8, 10% glucose, 5 mM EDTA, 0.1% bromophenol blue, 3% SDS, 2% , β-mercaptoethanol). In addition, proteins from lysates of 10⁷ transfected LMH cells (in 10 mM HEPES pH 7.5, 100 mM NaCl, 1 mM EDTA, 1% NP40) were immunoprecipitated using polyclonal rabbit anti-preS (D087) or anti-GFP (Clontech, Palo Alto, Ca., USA) antibodies and protein A sepharose. After washing, the pellet was dissolved in 50 µl protein-sample buffer. 25 µl of each lysate were separated by 10% SDS-PAGE, blotted to a PVDF membrane, immunostained with polyclonal antisera D084, D087 or D188 to DHBV proteins (D188 recognizing DHBV S-protein) or with polyclonal rabbit-anti-GFP antibody and visualized using the ECL-system (Amersham, Cleveland, Ohio, USA ).

Infectivity of recombinant virions was tested by infecting primary duck hepatocytes with rDHBV-S-GFP at different moi's (multiplicity of infection, measured as DNA-containing enveloped DHBV particles / primary cell) of 2 to 250 for 16 to 24 hours. Three days post infection, a faint green fluorescence became detectable, which increased markedly until day 5 and reached a maximum at day 8 post infection. The proportion of fluorescent cells was dependent on the multiplicity of infection used (see Figure 4). High multiplicities of infection of equal to or greater than 200 resulted in up to 90% of GFP positive cells.

Stable expression of the foreign gene was observed as long as viable hepatocytes could be kept in culture, with some variation in the intensity of green fluorescence between hepatocytes. Synthesis of DHBV core protein by rDHBV could be proven, as expected, by immunofluorescence costaining of GFP positive hepatocytes as well as by Western blot analysis of hepatocyte lysates. GFP-specific antibodies revealed two closely spaced bands of approximately 30 kDa, probably representing GFP and a DHBV-S/GFP-fusion. An additional RNA, initiating from the preS promoter, might serve for the expression of a preS/GFP fusion protein. However, no larger products corresponding to such a fusion protein were detected.

The number of fluorescent cells was strictly dose-dependent. As the recombinant viruses are replication-deficient, the percentage of GFP-positive cells can be taken as a measure for the infectious titre of incoming particles. At a multiplicity of infection of less than 5, only single cells showed a green fluorescence. Increasing moi's of 20 to 200 resulted in 5-10% to up to >90% of GFP positive hepatocytes. The intensity of green fluorescence varied between neighboring primary hepatocytes. Western blots of the cell lysates using anti-GFP antibodies showed again two immunoreactive bands around 30 kDa as in the transfected cells. These data demonstrate the efficient transfer and DHBV S-promoter controlled expression of a transgene by a recombinant hepadnavirus.

To test whether hepadnaviral vectors selectively target hepatocytes, cell-type specificity was analyzed *in vitro.* Primary hepatocyte cultures prepared by collagenase perfusion and differential sedimentation are known to contain 3 to 20% non-parenchymal liver cells, mainly sinusoidal endothelial cells and Kupffer cells (Johnston, D.E. & Jasuja, R. (1994) *Hepatology* 20:435-444). These can be distinguished from hepatocytes by their receptor-mediated uptake of acetylated LDL and by their ability to phagocytose (Irving, M. et al. (1984) *Gastroenterology* 87:1233-1247; McCuskey, R.S. et al. (1984) *Infect*. *Immun*. 45:278-280). None of these non-parenchymal cells, which accounted for some 15% of the total cell population in the cultures used in these experiments, showed GFP expression even after infection with rDHBV-GFP at a multiplicity of infection of 250-500. Similarly, no GFP expression was detectable when primary mouse hepatocytes (prepared as described in Example 3) were incubated with high multiplicity of infection rDHBV-GFP. These data indicate that delivery of the transgene by rDHBV is both hepatocyte- and species-specific.

To prove that rDHBV is suitable for liver-directed *in vivo* gene transfer, ducklings were infected at the day post hatching with 10⁹ rDHBV-GFP particles via intravenous injection. At day 7 post infection, fixed liver-tissue sections and isolated hepatocytes from these animals were analyzed by immunofluorescence microscopy. GFP-fluorescent hepatocytes were detectable in both specimens (1-GFP-positive cell per 10⁴ to 10⁵ hepatocytes) indicating successful *in vivo* gene transfer by rDHBV-GFP.

### Example 5: IFN Gene Transfer by Recombinant DHBV Interferes with the Establishment of DHBV Infection

IFN-α treatment is the current therapy of choice for chronic hepatitis B and hepatitis C. A homologous type I interferon has recently been cloned from ducks and addition of the recombinant protein to cultured fetal duck hepatocytes was shown to inhibit DHBV replication (Schultz *et al*., (1995) *Virology* 212:641-649). Therefore, duck IFN was chosen to test whether a potentially therapeutic gene could be delivered by a hepadnaviral vector, and whether the secretory protein was functional. Inclusion of the authentic duck IFN signal sequence would allow for IFN secretion, which then should exert similar effects on DHBV replication as the exogenously added cytokine.

Primary duck hepatocytes were co-infected with rDHBV-IFN, or rDHBV-GFP as a negative control, and with replication-competent, serum derived wildtype DHBV. As a positive control, IFN protein was added to wildtype DHBV infected hepatocytes at day 3 post infection at which time expression of IFN from rDHBV-IFN was expected to start. Progeny virus release into the cell culture medium, resulting from productive infection with wildtype DHBV, was monitored by DHBV-DNA dot blot analysis.

Untreated wildtype DHBV infected cells and cells co-infected with rDHBV-GFP produced equally high levels of progeny DHBV, as assessed by dot blot analysis. In contrast, approximately 20-fold less progeny DHBV (14- to 24-fold in different experiments) was released from cells coinfected with rDHBV-IFN. Similar reductions (16- to 25-fold) were obtained by treatment with recombinant IFN. Likewise, a strong suppression in the level of intracellular DHBV core- and L-protein was detected by Western blot analysis of cell lysates prepared at day 7 post infection. Figure 5 shows a quantitative evaluation of the time course of DHBV production (DHBV-DNA equivalents). These data demonstrate that a functional cytokine expressed after hepadnaviral gene-transfer interferes with establishment of an hepadnaviral infection *in vitro*.

### Example 6: Recombinant DHBV Superinfects DHBV-Infected Hepatocytes

For gene-therapeutic use in the treatment of chronic viral hepatitis, recombinant hepadnaviruses must be able to superinfect a liver with an established viral infection. To show that even superinfection of hepatocytes with a homologous virus is possible, primary hepatocytes were used from productively DHBV-infected ducks which all stained positive for DHBV S-protein, indicating productive DHBV infection. Incubation with rDHBV-GFP at moi's ranging from 25 to 100 resulted in 1-4% of GFP-positive hepatocytes (see Figure 6). Although this transduction efficiency is approximately 20-fold lower than the one observed with hepatocyte cultures not preinfected with DHBV, coexpression of GFP in S-protein positive cells proved that hepatocytes with an established wildtype DHBV infection were superinfected by rDHBV-GFP.

### Example 7: IFN Gene Transfer Suppresses an Established DHBV Infection

To test whether hepadnaviral cytokine gene transfer was principally suited for gene-therapy for chronic hepatitis B, DHBV-positive hepatocytes were superinfected with rDHBV-IFN and monitored for the release of progeny DHBV as described above. As shown in Fig. 7, DHBV production was decreased, relative to untreated controls, in a dose-dependent fashion, between 1.7 (multiplicity of infection of 25) and 4.5-fold (multiplicity of infection of 75), comparable to the effect observed by treatment with the cytokine protein at a dose showing maximal effect (4.1-fold reduction). No change in DHBV progeny production was seen upon superinfection with rDHBV-GFP, indicating that inhibition was caused by the transduced IFN gene.

### Example 8. Production of Recombinant HBV

As a basis for constructing recombinant hepatitis B virus genomes carrying the GFP gene, we used plasmid pCH-9/3091, which, upon transfection, give rise to the production of infectious HBV particles (Obert, S. et al. (1996) *EMBO J*. 15: 2565-2574) (Fig. 2). As with the construction of recombinant DHBV, care was taken to neither exceed the authentic genome size nor to affect cis-acting control elements (Ganem, D. (1996) in Hepadnaviridae: The Viruses and Their Replication, eds. Fields, B.N., Knipe, D.M., and Howley, P.M., Lippincott: Philadelphia, vol. 2: 2703-2737; Nassal, M. and Schaller, H. (1996) *J*. *Viral*. *Hepat*. 3:217-226); and Seeger, C. & Hu, J. (1997) *Trends in Microbiol.* 5: 447-450), and similarly to the findings of the DHBV experiment, only substitution of the small envelope (S) gene by foreign sequences (Fig. 2) turned out to be successful.

Plasmid pCH-S-GFP elicited strong GFP fluorescence 36 to 38 hours after transfection into appropriate hepatoma cells, demonstrating functional insertion of the foreign gene. Since S gene replacement destroys the surface protein and polymerase open reading frames, for generation of recombinant virus, the corresponding gene products were trans-complemented by cotransfection (Condreay, L.D. et al. (1990) *J*. *Virol.* 64:3249-3258; Schlicht, H.J. et al. (1989) *Cell* 56: 85-92) of encapsidation deficient helper construct pCH3142 (Schlicht, H.J. et al. (1989) *Cell* 56: 85-92, Bartenschlager, R. et al. (1990) *J*. *Virol*. 64: 5324-5332). This resulted in the production of enveloped recombinant HBV (rHBV) at titers between 10⁸ and 10⁹/ml. Virus could be concentrated up to 50-fold without loss of infectivity by polyethyleneglycol precipitation.

### Example 9: Successful Gene Transfer into Human Hepatocytes by Recombinant HBV Vectors

Infectivity of recombinant virus particles was demonstrated by incubating primary human hepatocytes with equal amounts of rHBV-GFP or wild-type HBV. One per 10² hepatocytes was found to be infected with either virus at day 6 post infection, utilizing specific immunofluorescence staining for HBV core protein as the assay for infected cells. It was assumed that infectivity of the recombinant virus is comparable to that of wild-type virus. One per 10⁴ hepatocytes showed clearly detectable GFP fluorescence, reaching its maximum at day 12 post infection. Due to the high autofluorescence background of human liver cells, weakly green fluorescent cells could not unequivocally be identified. Because of this technical limitation in GFP detection, assays detecting HBV core proteins were preferred for measurements of transduction efficiency.

### Example 10. Hepadnaviral Gene Transfer by HBV is Species and Hepatocyte-Specific

To test whether HBV vectors selectively target hepatocytes, we analyzed cell-type specificity *in vitro.* Incubation of duck hepatocytes with rHBV-GFP or of mouse hepatocytes with rHBV-GFP did not result in GFP expression. Combined with the data (Experiment 9) demonstrating that GFP is expressed in human hepatocytes infected with rHBV-GFP, these data indicate that delivery of the transgene by rHBV is species-specific. Combined with the data (Example 4) demonstrating that neither sinusoidal endothelial cells nor Kupffer cells expressed GFP, these data indicate that delivery of the transgene by hepadnaviral vectors is species and hepatocyte-specific.

## Claims

1. An in-vitro method for expressing a heterologous gene in hepatocytes comprising:
providing replication defective hepadnavirus particles at a titre level competent to infect hepatocytes, wherein a region of at least about 200 nucleotides of the S-gene of the hepadnavirus genome has been replaced with a heterologous gene of about 200 up to about 1200 nucleotides such that expression of the heterologous gene is regulated by regulatory sequences of the S-gene; and
infecting hepatocytes with the hepadnavirus such that the heterologous gene is delivered into the hepatocytes and expressed in the hepatocytes.

2. The method of claim 1, wherein the replication defective hepadnavirus particles are human hepatitis B virus particles.

3. The method of claim I, wherein the heterologous gene replaces the region of the S-gene such that nucleotides encoding at least one amino acid of the S protein are fused in-frame to the 5' end of the heterologous gene.

4. The method of claim 1, wherein the heterologous gene replaces the sequence of the S-gene.

5. The method of claim 1, wherein the heterologous gene is inserted after the authentic AUG triplet of the S-gene, and the heterologous gene is inserted such that nucleotides encoding at least one amino acid of the S protein are fused in-frame to the 5' end of the heterologous gene.

6. The method of claim 1, wherein the heterologous gene encodes a modulating agent.

7. The method of claim 6, wherein the modulating agent is a cytokine.

8. The method of claim 7, wherein the cytokine is selected from the group consisting of TNFα, IL-18, INFγ, IFNβ, and IFNα.

9. The method of claim 1, wherein the heterologous gene is a therapeutic gene.

10. A replication defective hepadnavirus particle, wherein a region of at least about 200 nucleotides of the S-gene of the hepadnavirus genome has been replaced with a therapeutic gene of about 200 up to about 1200 nucleotides such that expression of the therapeutic gene is regulated by regulatory sequences of the S-gene.

11. The replication defective hepadnavirus particle of claim 10, wherein the therapeutic gene encodes a cytokine.

12. The replication defective hepadnavirus particle of claim 11, wherein the cytokine is selected from the group consisting of TNFα, IFNβ, IL-18 and IFNγ and INFα.

13. A pharmaceutical composition comprising the replication defective hepadnavirus particle of claim 10 and a pharmaceutically acceptable carrier

14. A pharmaceutical composition comprising the replication defective hepadnavirus particle of claim 10 and a helper virus.

15. A method of producing therapeutic replication defective hepadnavirus particles at a titre level suitable for infecting hepatocytes comprising:
co-transfecting cells of a hepatoma cell line with:
(i) replication defective hepadnavirus constructs, wherein a region of at least about 200 nucleotides of the S-gene of the hepadnavirus DNA has been replaced with a therapeutic gene of about 200 up to about 1200 nucleotides regulated by regulatory sequences of the S-gene; and
(ii) a helper construct;
culturing these cells until infectious viral particles are produced; and recovering the infectious viral particles.

16. The method of claim 15, wherein the therapeutic gene encodes a cytokine.

17. The method of claim 15 or 16, wherein the hepatoma cell line is a helper cell line stably transfected with the helper construct.

18. An in-vitro method for producing replication defective recombinant hepadnavirus particles capable of expressing a heterologous gene of about 200 up to about 1200 nucleotides in hepatocytes comprising:
replacing at least about 200 nucleotides of the S-gene in a hepatitis B virus genome with the heterologous gene such that the expression of the heterologous gene is regulated by the S-promoter;
transcomplementing a replication deficient hepadnavirus by means of a helper plasmid that expresses the lacking hepadnaviral gene products;
infecting hepatocytes with the hepdnavirus, whereby the heterologous gene is delivered into the hepatocyte and expressed in the hepatocyte.

19. An rHBV genome, wherein at least about 200 nucleotides of an S-gene in the HBV genome are deleted and replaced by a heterologous gene of about 200 up to about 1200 nucleotides under control of the endogenous S promoter and wherein the sequences for reverse transcription are retained.

20. The rHBV genome of claim 19, wherein the heterologous gene encodes a cytokine.

21. The rHBV genome of claim 20, wherein the cytokine is selected from the group consisting of IFNα, IFNβ, IFNγ, TNFα, IL-18 or IL-12.

## Patentansprüche

1. Eine in-vitro Methode zur Expression eines heterologen Gens in Hepatozyten, die folgendes umfasst:
- Bereitstellung replikationsdefizienter hepadnaviraler Partikel, in denen eine Region von mindestens etwa 200 Nukleotiden des S-Gens des hepadnaviralen Genoms durch ein heterologes Gen von etwa 200 bis etwa 1200 Nukleotiden ersetzt ist, so dass die Expression des heterologen Gens unter Kontrolle regulatorischer Sequenzen des S-Gens steht, in einem Titer, der ausreicht, um Hepatozyten zu infizieren; und
- die Infektion von Hepatozyten mit dem Hepadnavirus, so dass das heterologe Gen in die Hepatoyzyten eingebracht wird und in den Hepatozyten exprimiert wird.

2. Die Methode nach Anspruch 1, wobei die replikationsdefekten hepadnaviralen Partikel humane Hepatitis B Virus Partikel sind.

3. Die Methode nach Anspruch 1, wobei das heterologe Gen so in das S-Gen eingefügt ist, dass Nukleotide, die für mindesten eine Aminosäure des S-Proteins kodieren, an das 5' Ende des Leserahmens des heterologen Gens fusioniert sind.

4. Die Methode nach Anspruch 1, wobei das heterologe Gen die Sequenz des S-Gens ersetzt.

5. Die Methode nach Anspruch 1, wobei das heterologe Gen nach dem authentischen AUG Triplet des S-Gens inseriert ist, und das heterologe Gen so inseriert ist, dass Nukleotide, die für mindesten eine Aminosäure des S-Proteins kodieren, an das 5' Ende des Leserahmens des heterologen Gens fusioniert sind.

6. Die Methode nach Anspruch 1, wobei das heterologe Gen für ein modulierendes Agens kodiert.

7. Die Methode nach Anspruch 6, wobei das modulierende Agens ein Zytokin ist.

8. Die Methode nach Anspruch 7, wobei der das modulierende Agens aus der Gruppe bestehend aus IFNβ, IFN-γ, TNFα, IL-18 and IL-12 ausgewählt ist.

9. Die Methode nach Anspruch 1, wobei das heterologe Gen ein therapeutisches Gen ist.

10. Ein replikationsdefizientes hepadnavirales Partikel, in dem eine Region von mindestens etwa 200 Nukleotiden des S-Gens des hepadnaviralen Genoms durch ein therapeutisches Gen von etwa 200 bis etwa 1200 Nukleotiden ersetzt ist, so dass die Expression des therapeutischen Gens unter Kontrolle regulatorischer Sequenzen des S-Gens steht.

11. Das replikationsdefiziente hepadnavirale Partikel nach Anspruch 10, bei dem das therapeutische Gen für ein Zytokin kodiert.

12. Das replikationsdefiziente hepadnavirale Partikel nach Anspruch 11, bei dem das Zytokin aus der Gruppe TNFα, IFNβ, IL-18 und IFN-γ und IFNα ausgewählt ist.

13. Eine pharmazeutische Zusammensetzung, die das replikationsdefiziente hepadnavirale Partikel nach Anspruch 10 und einen pharmazeutisch akzeptablen Träger umfasst.

14. Eine pharmazeutische Komposition, die das replikationsdefiziente hepadnavirale Partikel nach Anspruch 10 und einen Helfervirus umfasst.

15. Eine Methode zur Herstellung therapeutischer replikationsdefizienter hepadnaviraler Partikel in einem Titer, der ausreicht, um Hepatozyten zu infizieren, die folgendes umfasst:
- Ko-Transfektion von Zellen einer Hepatom-Zelllinie mit
(i) replikationsdefizienten hepadnaviralen Konstrukten, in denen eine Region von mindestens etwa 200 Nukleotiden des S-Gens des hepadnaviralen Genoms durch ein therapeutisches Gen von etwa 200 bis etwa 1200 Nukleotiden unter Kontrolle regulatorischer Sequenzen des S-Gens ersetzt ist; und
(ii) einem Helfer Konstrukt
- Kultivierung dieser Zellen, bis infektiöse virale Partikel produziert werden, und
- Aufreinigung der infektiösen viralen Partikel.

16. Die Methode nach Anspruch 15, wobei das therapeutische Gen für ein Zytokin kodiert.

17. Die Methode nach Anspruch 15 oder 16, wobei die Hepatom-Zelllinie eine Helfer-Zelllinie ist, die stabil mit einem Helfer-Konstrukt transfiziert ist.

18. Eine in-vitro Methode zur Herstellung replikationsdefizienter rekombinanter Hepadnavirus Partikel mit der Fähigkeit, ein heterologes Gen von etwa 200 bis etwa 1200 Nukleotiden in Hepatozyten zu exprimieren, die folgendes umfasst:
- das Ersetzen von mindestens etwa 200 Nukleotiden des S-Gens in einem Hepatitis B Virus Genom durch ein heterologes Gen, so dass die Expression des heterologen Gens durch den S-Promotor reguliert ist;
- Transkomplementation eines replikationsdefizienten Hepadnavirus mit Hilfe eines Helfer-Plasmids, dass die fehlenden hepadnaviralen Genprodukte exprimiert;
- Infektion von Hepatozyten mit dem Hepadnavirus, wobei das heterologe Gen in die Hepatozyten eingebracht und in den Hepatozyten exprimiert wird.

19. Ein rHBV Genom, in dem mindestens etwa 200 Nukleotide eines S-Gens im HBV Genom deletiert und durch ein heterologes Gen von etwa 200 bis zu etwa 1200 Nukleotiden unter Kontrolle des endogenen S Promotors ersetzt sind, und in dem die Sequenzen, die für die reverse Transkription notwendig sind, erhalten sind

20. Das rHBV Genom nach Anspruch 19, wobei das heterologe Gen für ein Zytokin kodiert.

21. Das rHBV Genom nach Anspruch 20, wobei das Zytokin aus der Gruppe bestehend aus IFNα, IFNβ, IFN-γ, TNFα, IL-18 oder IL-12 ausgewählt ist.

## Revendications

1. Méthode in-vitro d'expression d'un gène hétérologue dans des hépatocytes, comprenant :
- la fourniture de particules de l'hépadnavirus incapables de réplication à un titre capable d'infecter des hépatocytes, une région d'au moins environ 200 nucléotides du gène S du génome de l'hépadnavirus étant remplacée par un gène hétérologue d'une longueur comprise entre environ 200 et environ 1 200 nucléotides de sorte que l'expression du gène hétérologue est régulée par des séquences régulatrices du gène S ; et
- l'infection d'hépatocytes avec l'hépadnavirus de sorte que le gène hétérologue est délivré dans les hépatocytes et exprimé dans les hépatocytes.

2. Méthode selon la revendication 1, **caractérisée en ce que** les particules d'hépadnavirus incapables de réplication sont des particules du virus de l'hépatite B humain.

3. Méthode selon la revendication 1, **caractérisée en ce que** le gène hétérologue remplace la région du gène S de sorte que des nucléotides codant au moins un acide aminé de la protéine S sont fusionnés avec le cadre à l'extrémité 5' du gène hétérologue.

4. Méthode selon la revendication 1, **caractérisée en ce que** le gène hétérologue remplace la séquence du gène S.

5. Méthode selon la revendication 1, **caractérisée en ce que** le gène hétérologue est inséré après le triplet AUG authentique du gène S, et le gène hétérologue est inséré de telle sorte que des nucléotides codant au moins un acide aminé de la protéine S sont fusionnés dans le cadre à l'extrémité 5' du gène hétérologue.

6. Méthode selon la revendication 1, **caractérisée en ce que** le gène hétérologue code un agent de modulation.

7. Méthode selon la revendication 6, **caractérisée en ce que** l'agent de modulation est une cytokine.

8. Méthode selon la revendication 7, **caractérisée en ce que** la cytokine est sélectionnée dans le groupe constitué de TNFα, IL-18, INFγ, IFNβ et IFNα.

9. Méthode selon la revendication 1, **caractérisée en ce que** le gène hétérologue est un gène thérapeutique.

10. Particule de l'hépadnavirus incapable de réplication, **caractérisée en ce qu'**une région d'au moins environ 200 nucléotides du gène S du génome de l'hépadnavirus a été remplacée par un gène thérapeutique de longueur comprise entre environ 200 et environ 1 200 nucléotides de sorte que l'expression du gène thérapeutique est régulé par des séquences régulatrices du gène S.

11. Particule de l'hépadnavirus incapable de réplication selon la revendication 10, **caractérisée en ce que** le gène thérapeutique code une cytokine.

12. Particule de l'hépadnavirus incapable de réplication selon la revendication 11, **caractérisée en ce que** la cytokine est sélectionnée dans le groupe constitué de TNFα, IFNβ, IL-18 et INFγ et IFNα.

13. Composition pharmaceutique comprenant la particule d'hépadnavirus incapable de réplication de la revendication 10 et un support pharmaceutique acceptable.

14. Composition pharmaceutique comprenant la particule d'hépadnavirus incapable de réplication de la revendication 10 et un virus auxiliaire.

15. Méthode de production de particules d'hépadnavirus thérapeutiques incapables de réplication à un titre capable d'infecter des hépatocytes, comprenant :
la co-transfection de cellules d'une ligne de cellules hépatomes avec
(i) réplication de structures d'hépadnavirus défectueuses, une région d'au moins environ 200 nucléotides du gène S de l'ADN de l'hépadnavirus ayant été remplacée par un gène thérapeutique d'une longueur comprise entre environ 200 et environ 1 200 nucléotides régulée par des séquences régulatrices du gène S ; et
(ii) une structure assistante
la culture de ces cellules jusqu'à ce que des particules virales infectieuses soient produites ; et
la récupération des particules virales infectieuses.

16. Méthode selon la revendication 15, **caractérisée en ce que** le gène thérapeutique code pour une cytokine.

17. Méthode selon la revendication 15 ou 16, **caractérisée en ce que** la ligne cellulaire d'hépatomes est une ligne cellulaire assistante transfectée de façon stable avec une structure assistante.

18. Méthode in-vitro pour produire des particules d'hépadnavirus recombinantes incapables de réplication, capables d'exprimer dans les hépatocytes un gène hétérologue d'une longueur comprise entre environ 200 et environ 1 200 nucléotides, comprenant :
le remplacement d'au moins environ 200 nucléotides du gène S dans le génome d'un virus de l'hépatite B par le gène hétérologue de sorte que l'expression du gène hétérologue soit régulé par le promoteur S ;
la transcomplémentation d'un hépadnavirus incapable de réplication au moyen d'un plasmide assistant qui exprime le manque de produits du gène hépadnaviral ;
l'infection des hépatocytes avec l'hépadnavirus, le gène hétérologue étant délivré dans l'hépatocyte et exprimé dans l'hépatocyte.

19. Un génome rHBV, **caractérisé en ce que** au moins environ 200 nucléotides du gène S dans le génome HBV sont enlevé et remplacés par un gène hétérologue d'une longueur comprise entre environ 200 et environ 1 200 nucléotides sous contrôle du promoteur S endogène, et **en ce que** les séquences pour la transcription inverse sont conservées.

20. Génome rHBV selon la revendication 19, **caractérisé en ce que** le gène hétérologue code pour une cytokine.

21. Génome rHBV selon la revendication 20, **caractérisé en ce que** la cytokine est sélection dans le groupe constitué par IFNα, IFNβ, INFγ, TNFα, IL-18 ou IL-12.
